Europäisches Patentamt

European Patent Office

Office européen des brevets

(11) Publication number: **0 078 957**
**B1**

# EUROPEAN PATENT SPECIFICATION

(45) Date of publication of patent specification: **26.03.86**

(51) Int. Cl.⁴: **A 61 B 1/04**

(21) Application number: **82109785.4**

(22) Date of filing: **22.10.82**

(54) **Endoscopic photographing apparatus.**

(30) Priority: **31.10.81 JP 175279/81**

(43) Date of publication of application:
**18.05.83 Bulletin 83/20**

(45) Publication of the grant of the patent:
**26.03.86 Bulletin 86/13**

(84) Designated Contracting States:
**AT BE CH DE FR GB IT LI NL SE**

(56) References cited:
**EP-A-0 036 196**
**DE-B-1 766 328**
**GB-A-2 065 313**
**US-A-3 599 630**

(73) Proprietor: **OLYMPUS OPTICAL CO., LTD.**
**43-2, 2-chome, Hatagaya Shibuya-ku**
**Tokyo 151 (JP)**

(72) Inventor: **Matsui, Koichi**
**2-13-3-350, Wakagi**
**Itabashi-ku Tokyo (JP)**

(74) Representative: **KUHNEN & WACKER**
**Patentanwaltsbüro**
**Schneggstrasse 3-5 Postfach 1729**
**D-8050 Freising (DE)**

Courier Press, Leamington Spa, England.

# Description

This invention relates to an endoscopic photographing apparatus which is adapted for the automatic control of exposure.

An endoscopic photographing apparatus generally comprises an endoscope body, a camera and a light-supplying unit. The camera used is normally of the exclusive type. However, the ordinary single lens reflex camera may be applied for this purpose. No diaphragm mechanism is provided on the camera side of an endoscopic photographing apparatus. When a body cavity is photographed, exposure is not controlled by means of a camera shutter, but by controlling the emission of light from the light-supplying unit, because the body cavity or coeliac cavity, respectively is completely dark. The light-supplying unit is provided with a diaphragm mechanism to control the emission of light. This diaphragm mechanism is manually operated at the time of observation intended for framing. Generally, a larger amount of light is required for photographing than for observation, presenting difficulties in defining an optimum aperture scale at the time of photographing. Hitherto, therefore, endoscopic photographing has been carried out with the stop mechanism left open. When an amount of light entering a film during photographing reaches a prescribed level, then the light-supplying unit ceases to be operated, thereby enabling photographing to be effected with proper exposure.

An example for such an endoscopic photographing apparatus is described in US—PS 3,599,630. Apart from the relative complicated mechanical structure of this known endoscopic photographing apparatus, with the improvement of endoscopic surgery the following problem has appeared:

When the amount of light entering the film during photographing reaches the prescribed level and light emission from the light-supplying unit is terminated, the response speed of an electric circuit imposes a limit on the shortest period of exposure. In the endoscopic photographing of the coeliac cavity, only the light emitted from the light-supplying unit to illuminate a foreground subject through a light guide defines the brightness of the foreground subject. For this reason, the shorter the distance between the eyepiece section of the endoscope body and the foreground subject, the more increased the brightness of the foreground subject will be. Therefore, at the time of close photographing, for example, the photographing of a small object, the period of exposure is sometimes demanded to be shorter than the shortest possible period which is decided by the response speed of the electric circuit. Thus, an undesirable event of excess exposure occurs in the above mentioned case.

It is therefore the object of the present invention to provide an endoscopic photographing apparatus being capable of properly controlling film exposure even in the event of close photographing inside the coeliac cavity.

Solution of this object is achieved by the characterizing features of claim 1.

This invention can be more fully understood from the following detailed description when taken in conjunction with the accompanying drawings, in which:

Figure 1 is a prospective view of an embodiment of an endoscopic photographing apparatus according to invention;

Figure 2 is a block diagram showing the interior of the endoscopic photographing apparatus shown in Figure 1;

Figure 3 is a perspective view of a diaphragm mechanism included in the endoscopic photographing apparatus shown in Figure 1; and

Figure 4 shows the block diagram of a diaphragm mechanism-controlling circuit.

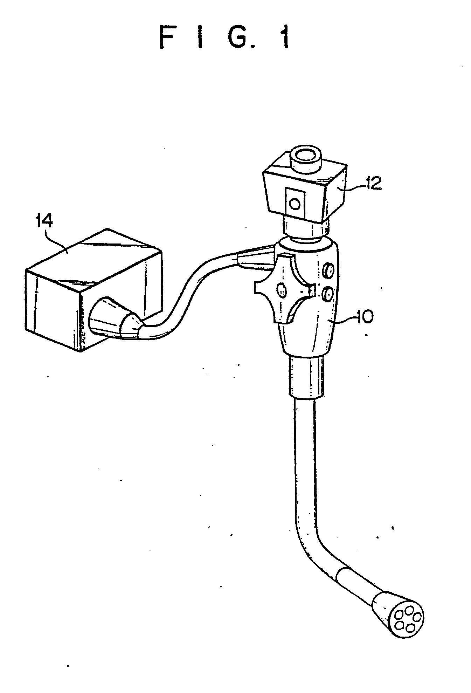

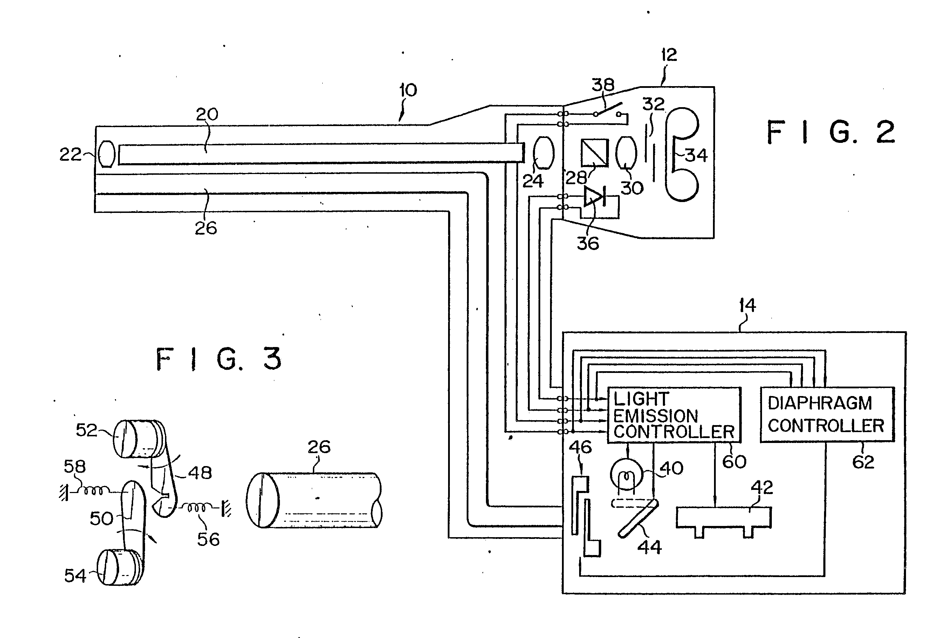

Description is now given with reference to the accompanying drawings of an embodiment of an endoscopic photographing apparatus according to the invention. In Figure 1, a camera 12 and light-supplying unit 14 are connected to an endoscope 10 comprising an image guide and a light guide. The camera 12 is connected to the eyepiece section of the endoscope 10 to photograph a foreground subject through the image guide. The camera 12 is often of an exclusive type which is freed of a diaphragm mechanism. The ordinary single lens reflex camera may be applied for this purpose. The light-supplying unit 14 is connected to the proximal end of the light guide to the eyepiece side thereof to illuminate a foreground subject through the light guide. Figure 2 is a block diagram showing the interior of the endoscopic photographing apparatus. An image guide 20 included in the endoscope 10 extends between the objective section and eyepiece section of the endoscope 10. The objective section is fitted with an objective lens 22, and the eyepiece section is provided with an eyepiece lens 24. A light guide 26 is connected at one end to the objective section, and at the other end to the output port of the light-supplying unit 14. The light entering the camera 12 is divided into two portions by a half prism 28. One portion of the light is carried to a film 34 through a lens 30 and shutter 32. The other portion of the light is conducted to a photodiode 36. The camera 12 is also fitted with a synchronizing switch 38 operated in synchronization with the actuation of a shutter release. The light-supplying unit 14 comprises an observation iodine lamp 40 and photographing flash discharge tube 42. A mirror 44 selectively supplies the output lights from the iodine lamp 40 and flash discharge tube 42 to the light guide 26. A diaphragm mechanism 46 is interposed between the mirror 44 and the output port of the light-supplying unit 14.

Description is now given with reference to the perspective view of Figure 3 of the details of the diaphragm mechanism 46. This diaphragm mechanism 46 includes a diaphragm 48 provided with a wedge-shaped notch and another diaphragm 50 and the corresponding solenoids 52 and 54. The diaphragms 48 and 50 are respectively rotated by the corresponding solenoids 52 and 54 in the opposite directions indicated by the arrows in a state superposed on each other in such a manner that the opening of the wedge-shaped notch of the diaphragm 48 is substantially narrowed. Normally, the diaphragms 48 and 50 are so urged by the corresponding springs 56 and 58 as to substantially broaden the opening of the wedge-shaped notch. When the solenoids 52 and 54 are rendered nonconducting, the notch opening is left intact.

Referring again to Figure 2, the operation of the iodine lamp 40, flash discharge tube 42 and mirror 44 is controlled by a light emission controller 60. The operation of the diaphragm mechanism 46 is controlled by a diaphragm controller 62. Output signals from the photodiode 36 and synchronizing switch 38 held in the camera 12 are supplied to both light emission controller 60 and diaphragm controller 62. Detailed description is omitted of the already known light emission controller 60. This light emission controller 60 well serves the purpose, provided it allows for the following operations: at the time of observation the iodine lamp 40 emits light; the light sent forth from the iodine lamp 40 is reflected by the mirror 44 and then enters the light guide 26; when the synchronizing switch 38 issues a synchronizing signal, then the flash discharge tube 42 emits light; the mirror 44 is lifted to a position indicated by a broken line shown in Figure 2; and consequently the light emitted from the flash discharge tube 42 enters the light guide 26. An output signal from the photodiode 36 is integrated upon receipt of the synchronizing signal. When the integrated value reaches a prescribed level corresponding to an exposure constant related to, for example, film sensitivity, then the flash discharge tube 42 ceases to emit light.

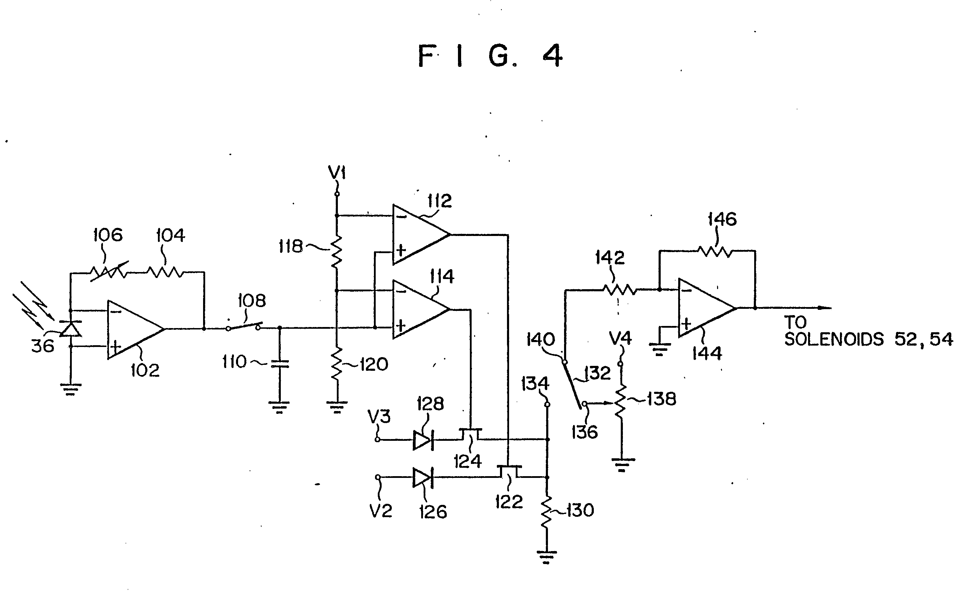

Detailed description is now given with reference to Figure 4 of the circuit arrangement of the diaphragm controller 62. The anode and cathode of the photodiode 36 are respectively connected to noninverting and inverting input terminals of an operational amplifier 102. The noninverting input terminal is grounded, and an invariable resistor 104 and a variable resistor 106 are connected in series between the output terminal and noninverting input terminal of the operational amplifier 102. The resistance of the variable resistor 106 varies in accordance with the rotation of the diaphragms 48 and 50. The greater the extent to which the diaphragms 48 and 50 are superposed on each other, the higher the resistance of the variable resistor 106. As a result, the amplification factor of the operational amplifier 102 increases. Now let it is assumed that a foreground subject has a constant brightness. If, under such conduction, the diaphragms 48 and 50 are superposed to a greater extent, then the output signal from the photodiode 36 becomes a low voltage level. Eventually, therefore, the voltage level of the output terminal of the operational amplifier 102 remains constant regardless of the aperture scale. The constant level corresponds to the level when the observation light has a maximum brightness. An output signal from the operational amplifier 102 is conducted through a switch 108 to one end of a capacitor 110 and noninverting input terminals of comparators 112 and 114. The switch 108 is controlled by a signal delivered from the synchronizing switch 38. The capacitor 110 is grounded at the other end. First power source V1 is grounded through a series circuit of resistors 118 and 120. The power source V1 and the junction of the resistors 118 and 120 are respectively connected to the inverting input terminals of the comparators 112 and 114. The voltage of a first power source V1 is set at a level corresponding to the film sensitivity ASA and the shortest length of time T for which light is to be issued from the flash discharge tube 42

$$(V1 = \frac{K}{T \times ASA}; \ K = constant).$$

The output terminals of comparators 112 and 114 are respectively connected to the gates of the corresponding analog switches 122 and 124. Second and third power sources V2 and V3 are respectively connected to the analog switches 122 and 124 at one end through corresponding diodes 126 and 128. In this case, the second power source V2 is chosen to have a more positive voltage than the third power source V3. The other ends of the analog switches 1/2 and 124 are grounded through a resistor 130 and connected to a first contact 134 of a switch 132. A second contact 136 of the switch 132 is connected to the output terminal of a potentiometer 138, which is connected between a fourth voltage source V4 and ground. The operation of the switch 132 is changed over upon receipt of a synchronizing signal. The movable contact 140 of the switch 132 is connected to the inverting input terminal of an operational amplifier 144 through an input resistor 142. The noninverting input terminal of the operational amplifier 144 is grounded. A feed back resistor 146 is connected between the output terminal and inverting input terminal of the operational amplifier 144. The output terminal of the operational amplifier 144 is connected to the rotary solenoids 52 and 54 to act as the output terminal of the diaphragm controller 62.

Description is now given of the operation of an endoscopic photographing apparatus embodying this invention. The endoscope 10, camera 12, and light-supplying unit 14 are connected together as shown in Figure 1. In this state, the objective section of the endoscope is carried into the coeliac cavity. At this time, the mirror 44 occupies the position indicated by a solid line of Figure 2. The iodine lamp 40 emits light, which in turn illuminates a foreground subject through the light guide 26. The switches 108 and 132 of the diaphragm controller 62 remain in the state indicated in Figure 4. The photographer looks at the affected spot of the coeliac cavity of a patient through the finder of the camera 12. An excess or insufficient amount of light is emitted depending on the position taken by the defective spot. To avoid such irregularity of illumination, therefore, the potentiometer 138 is operated to change the level of voltage impressed on the operational amplifier 144 and also the level of voltage supplied to the rotary solenoids 52 and 54. In other words, the aperture scale of the diaphragms 48 and 50 is varied according to the level of output voltage from the potentiometer 138. The diaphragms 48 and 50 are brought to rest at a spot where a balance is attained between the torque of the solenoids 52 and 54 and the urging force of the springs 56 and 58. Since the springs 56 and 58 urge the diaphragms 48 and 50 in the direction in which they are allowed to open, a lower output voltage from the potentiometer 138 more broadens the diaphragm opening.

The light emitted from the lamp 40 through the properly controlled diaphragm opening illuminates the affected spot of the coeliac cavity of a patient. The light reflected from the foreground subject is transmitted through the image guide 20 to the camera 12 and then to the photodiode 36. An output signal from the photodiode 36 is amplified by the operational amplifier 102. The variable resistor 106 changes in resistivity, as previously described, according to the extent to which the diaphragms 48 and 50 are rotated. When the diaphragms 48 and 50 are fully open, the resistivity of the variable resistor 106 is reduced to zero. The invariable resistor 104 is provided as the feedback resistor of the operational amplifier 102. Therefore, even when the diaphragm opening is appreciably narrowed, an output signal from the amplifier 102 has a level corresponding to the brightness of a reflected observation light when the diaphragms are left open. At this time the switch 108 is closed, causing an output signal from the operational amplifier 102 to be supplied to the comparators 112 and 114. Description is now given of the case where the output signals from the comparators 112 and 114 have a logic level "0" alike. In this case, it is anticipated that the operational amplifier 102 will produce an output signal having a low voltage level and the time of exposure will be correspondingly extended. In such case, the analog switches 122 and 124 are rendered nonconducting alike, causing the first contact 134 of the switch 132 to be grounded. Description is now given of the case where an output signal from the comparator 112 has a logic level "0", and an output signal from the comparator 114 has a logic level "1". In such case, it is anticipated that a larger amount of a reflected light is received than in the aforementioned case, causing the time of exposure to be correspondingly shortened. At this time, the first power source V1 has a voltage level corresponding to the controllable shortest time of exposure. If, in such case, an output signal from the comparator 114 has a logic level "1" and the diaphragms 48 and 50 are left open, then it means that a time of exposure shorter than the controllable shortest exposure time is optimum. At this time, the analog switch 124 is rendered conducting, causing the first contact 134 of the switch 132 to be connected to the third power source V3. When an output signal from the operational amplifier 102 has a higher voltage level, then output signals from the comparators 112 and 114 have a logic level "1" alike. This is the case where a larger amount of a reflected light is received than in the aforementioned two cases, and the exposure time is extremely shortened. In such case, the analog switches 122 and 124 are rendered conducting alike, and the diodes 126 and 128 cooperate to cause the first contact 134 of the switch 132 to be connected to the second power source V2 (having a higher voltage level than the third power source V3).

As described above, the first contact 134 of the switch 132 is chosen to have a voltage level corresponding to an amount of the light reflected from a foreground subject in the case a prescribed amount of the observation light is emitted. When a small amount of the reflected light is received, then the first contact 134 is not supplied with any voltage. When a medium amount of the reflected light is received, then the first contact 134 has a voltage level V3. When a large amount of the reflected light is received when the first contact 134 has a voltage V2.

When the release button (not shown) of the camera is depressed after the frame of a foreground subject to be photographed is determined, then the shutter 32 is opened, and simultaneously the release switch 38 is closed. When a synchronizing signal is supplied from the release switch 38 to the light emission controller 60, then the lamp 40 is extinguished, and the flash discharging tube 42 emits light, causing the mirror 44 to be shifted to a position indicated by the broken line of Figure 2. As a result, a flash is carried into the light guide 26, thereby enabling the film to be exposed to the light. When a synchronizing signal is supplied to the diaphragm controller 62, the switch 108 is rendered nonconducting. Accordingly, the switch 132 is now operated through the first contact 134. After the start of photographing, the light-supplying unit 14 emits a larger amount of light. At this time, the switch 108 is rendered nonconducting, causing the amount of light reflected from a foreground subject immediately before photographing to be stored in the capacitor 110. As a result, the voltage impressed on the first contact of the switch 132 is retained at the level which prevailed immediately before the start of photographing. As the switch 132 is now operated through the first contact 134, the voltage impressed on the solenoids 52 and 54 has a level corresponding to the amount of the received reflected light. When a small amount of the reflected light is received, no voltage is impressed on the solenoids 52 and 54, which therefore are not actuated. As a result, the diaphragms 48 and 50 are left open by the urging force of the springs 56 and 58. As the amount of the reflected light increases, the solenoids 52 and 54 are impressed with the voltage V3 or V2. Therefore, the diaphragms 48 and 50 are superposed on each other to a greater extent against the urging force of the springs 56 and 58. As a result, the light guide receives only that amount of light which corresponds to the brightness of a foreground subject measured immediately before photographing.

In the light emission controller 60, an output signal from the photodiode 36 is integrated. When the integrated value of the signal reaches a prescribed level corresponding to the film sensitivity, then the light emission controller 60 prevents the flash discharge tube 42 from emitting light. Thus, the exposure of the film is brought to an end. Since, at close photographing, an amount of light carried into the light guide 26 decreases, the control of exposure time, that is, the control of the operation of the flash discharge tube 42 is effected to the extent which falls well within the range of the response time of the related circuit. Therefore, excess exposure is avoided. In the foregoing description, two comparators are provided to judge the brightness of a foreground subject immediately before photographing. When, however, the diaphragms 48 and 50 are operated only in two stages, a single comparator well serves the purpose. Conversely where a larger number of comparators are applied, diaphragming can be controlled more precisely. The diaphragm mechanism is not limited to the aforementioned arrangement. Further it is not necessary to provide separate lamps for photographing and observation. In short, the primary object of this invention is to reduce the diaphragm opening if a foreground subject is illuminated by an excessively bright light at the time of photographing and apply a longer exposure time than the controllable shortest response time of the related circuit.

## Claims

1. An endoscopic photographing apparatus which comprises:

an endoscope (10) having a light guide (26) and an image guide (20);

a camera (12) which is connected to an eyepiece section of said endoscope (10), receives light reflected from a foreground subject through the image guide (20), and is further provided with an element (36) for effecting the photoelectric conversion of light entering a film (34);

a light-supplying unit (14) which is connected to the proximal end of said endoscope light guide (26) to emit light into the light guide (26) and is further provided with light-emitting means (40, 42) for emitting observation—and photographing light into the light guide (26) and a diaphragm mechanism (46) interposed between the light-emitting means (40, 42) and the light guide (26);

a light emission controller (60) which is actuated at the release of the camera shutter to cause the light-emitting means (42) to send forth photographing light for a prescribed length of time and wherein said light emission controller (60) begins to integrate the output signal from the photo-electric conversion element (36) from the start of a photographing cycle, and, when the integrated value reaches a prescribed level, ensures that no more photographing light reaches the light guide, characterized by further comprising a diaphragm controller (62) for setting, immediately before the start of the photographing cycle, the aperture size of said diaphragm mechanism (46) to a value corresponding to the output signal from the photo-electric conversion element (36).

2. An endoscopic photographing apparatus according to claim 1, characterized in that said diaphragm controller (62) comprises a comparator (112, 114) for comparing an output signal from the photo-electric conversion element (36) with a signal denoting the amount of light received from a foreground subject when the light-emitting means (42) sends forth light for the shortest possible period, and the diaphragm scale is determined corresponding to the output signal from the comparator (112, 114).

3. An endoscopic photographing apparatus according to claim 2, characterized in that said diaphragm mechanism (46) comprises a plurality of diaphragms (48, 50) which are so urged as to cause the diaphragm opening to be changed in a first direction by springs (56, 58) and in a second direction by the rotation of rotary solenoids (52, 54); and said diaphragm controller (62) applies different levels of voltage (V2, V3) on the rotary solenoids (52, 54) during photographing through switches (122, 124) whose conductions are controlled according to the output signal from the comparator (112, 114).

4. An endoscopic photographing apparatus according to claim 2 or 3, characterized in that said light-supplying unit (14) comprises a first light-emitting element (40) for emitting the observation light before photographing and a second light-emitting element (42) for emitting the photographing light during photographing; and said diaphragm controller (62) comprises a capacitor (10) for storing immediately before the start of the photographing cycle an output signal which is issued from the photo-electric conversion element (36) upon receipt of light from the first light-emitting element (40); and the signal held in the capacitor (110) is supplied to the comparator (112, 114).

## Patentansprüche

1. Endoskopische Fotografiereinrichtung, mit einem Endoskop (10), welches einen Lichtleiter (26) und einen Bildleiter (20) aufweist;

mit einer Kamera (12), welche mit einem Beobachtungsabschnitt an dem Endoskop (10) verbunden ist und Licht empfängt, welches von eine Objekt durch den Bildleiter (20) reflektiert wird und weiterhin ein Element (36) aufweist, welches eine fotoelektrische Umwandlung des Lichtes bewirkt, welches auf einen Film (34) auftrifft;

mit einer Lichtzufuhr-Vorrichtung (14), welche mit dem proximalen Ende des endoskopischen Lichtleiters (26) verbunden ist, um Licht in den Lichtleiter (26) zu emittieren und welche weiterhin eine lichtemittierende Einrichtung (40, 42) aufweist, um Beobachtungs- und Fotografierlicht in den Lichtleiter (26) zu emittieren, sowei einen Blendenmechanismus (46), der zwischen der lichtemittierenen Einrichtung (40, 42) und dem Lichtleiter (26) angeordnet ist; und

mit einer Lichtemissions-Steuerung (60) welche beim Freigeben des Kameraverschlusses betätigt wird, und die lichtemittierende Einrichtung (42) veranlaßt, Fotografierlicht während einer festgelegten Zeitdauer zu emittieren, wobei die Lichtemissions-Steuerung (60) zu Beginn eines Fotografierzyklus mit dem Aufintegrieren des Ausgangssignales von dem fotoelektrischen Wandlerelement (36) beginnt und, wenn der aufintegrierte Wert einen festgelegten Wert erreicht, sicherstellt, daß kein Fotografierlicht mehr in den Lichtleiter eintritt, gekennzeichnet durch

eine Blendensteuerung (62) zum Festsetzen der Öffnungsgröße des Blendenmechanismus (46) auf einen Wert entsprechend des Ausgangssignales von dem fotoelektrischen Wandelelement (36) umittelbar vor dem Beginn des Fotografierzyklus.

2. Fotografiereinrichtung nach Anspruch 1, dadurch gekennzeichnet, daß die Blendensteuerung (62) einen Komparator (112, 114) aufweist, um ein Ausgangssignal von dem fotoelektrischen Wandlerelement (36) mit einem Signal zu vergleichen, welches die Lichtmenge anzeigt, die von einem zu fotografierenden Objekt empfangen wird, wenn die lichtemittierende Einrichtung (42) Licht für die kürzest mögliche Zeitdauer emittiert, wobei die Blendengröße entsprechend dem Ausgangssignal von dem Komparator (112, 114) festgelegt wird.

3. Fotografiereinrichtung nach Anspruch 2, dadurch gekennzeichnet, daß der Blendenmechanismus (46) eine Mehrzahl von Blenden (48, 50) aufweist, die derart vorgespannt sind, daß die Blendenöffnung in einer erste Richtung mittels Federn (56, 58) änderbar ist und in eine zweite Richtung durch die Drehung von Drehmagneten (52, 54) änderbar ist; und daß die Blendensteuerung (62) verschiedene Spannungswert (V2, V3) den Drehmagneten (52, 54) über Schalter (122, 124) zuführt, deren Schaltzustände in Abhängigkeit von dem Ausgangssignal des Komparators (112, 114) gesteuert werden.

4. Fotografiereinrichtung nach Anspruch 2 oder 3, dadurch gekennzeichnet, daß die Lichtzufuhr-Vorrichtung (14) ein erstes lichtemittierendes Element (40) zur Abgabe von Beobachtungslicht vor dem Fotografiervorgang und ein zweites lichtemittierendes Element (42) aufweist, um während des Fotografiervorganges ein Fotografierlicht zu emittieren; und daß die Blendensteuerung (62) einen Kondensator (10) aufweist, um unmittelbar vor dem Beginn des Fotografierzyklus ein Ausgangssignal zu speichern, welches von dem fotoelektrischen Wandlerelement (36) beim Empfang des Lichtes von dem ersten lichtemittierenden Element (42) erzeugt wird, wobei das Signal in dem Kondensator (110) dem Komparator (112, 114) zugeführt wird.

**Revendications**

1. Appareil photographique endoscopique comprenant:

un endoscope (10) équipé d'un guide (21) de lumière et d'un guide (20) d'image;

un appareil de prise de vue (12) relié à une partie oculaire de l'endoscope (10), recevant de la lumière réfléchie par un sujet disposé à l'avant-plan, par l'intermédiaire du guide (20) d'image et équipé en outre d'un élément (36) pour effectuer la conversion photoélectrique de la lumière frappant un film (34);

un dispositif d'éclairage (14), relié à l'extrémité la plus proche du guide (26) de lumière de l'endoscope, pour envoyer de la lumière dans le guide (26) de lumière et doté en outre d'un moyen (40, 42) pour émettre une lumière d'observation et de photographie dans le guide (26) de lumière et d'un mécanisme (46) à diaphragme interposé entre le moyen (40, 42) émetteur de lumière et le guide (26) de lumière;

un régulateur (60) d'émission de lumière qui est actionné au moment où se déclenche l'obturateur de l'appareil de prise de vue afin de provoquer la projection, pendant une durée de temps prédéterminée, de lumière photographique par le moyen (42) émetteur de lumière, cependant que le régulateur (60) d'émission de lumière commence à intégrer le signal de sortie émis par l'élément (36) de conversion photoélectrique dès le début d'un cycle photographique et, lorsque la valeur intégrée atteint un niveau prédéterminé, s'assure qu'aucune lumière de photographie n'atteint plus le guide de lumière, caractérisé en ce qu'il comprend en outre un régulateur (62) de diaphragme pour régler, immédiatement avant le début du cycle photographique, l'ouverture du mécanisme (46) à diaphragme à une valeur correspondant au signal de sortie émis par l'élément (36) de conversion photoélectrique.

2. Appareil photographique endoscopique suivant la revendication 1, caractérisé en ce que le régulateur (62) du diaphragme comprend un comparateur (112, 114) pour comparer un signal de sortie de l'élément (36) de conversion photoélectrique, à un signal indiquant la quantité de lumière reçue d'un sujet situé à l'avant-plan lorsque le dispositif (42) émetteur de lumière envoie de la lumière pendant une durée aussi courte que possible, et en ce que l'échelle de réglage du diaphragme est déterminée en fonction du signal de sortie du comparateur (112, 114).

3. Appareil photographique endoscopique suivant la revendication 2, caractérisé en ce que le mécanisme (46) à diaphragme comprend une pluralité de diaphragme (48, 50) qui sont commandés de façon telle que l'ouverture du diaphragme est modifiée dans un premier sens par des ressorts (56, 58) et dans un deuxième sens par la rotation de solénoïdes rotatifs (52, 54) et en ce que le régulateur (62) du diaphragme applique différents niveaux de tension (V2, V3) aux solénoïdes rotatifs (52, 54) pendant la photographie, au moyen de commutateurs (122, 124) dont les conductions sont réglées en fonction du signal de sortie du comparateur (112, 114).

4. Appareil photographique endoscopique suivant l'une des revendications 2 ou 3, caractérisé en ce que le dispositif (14) d'éclairage comprend un premier élément (40) émetteur de lumière pour émettre la lumière d'observation avant la photographie et un deuxième élément (42) émetteur de lumière pour émettre la lumière de photographie pendant la photographie; et en ce que le régulateur (62) du diaphragme comprend un condensateur (110) ayant pour rôle de mettre en mémoire, immédiatement avant la début du cycle de photographie, un signal de sortie de l'élément (36) de conversion photoélectrique lors de la réception de la lumière venant du premier élément (40) émetteur de lumière; et en ce que le signal mémorisé par le condensateur (110) est appliqué au comparateur (112, 114).

# F I G. 1

F I G. 2

F I G. 3

LIGHT EMISSION CONTROLLER

DIAPHRAGM CONTROLLER

0 078 957

F I G. 4

TO
SOLENOIDS 52, 54

0 078 957